# EUROPEAN PATENT APPLICATION

(11) **EP 1 808 118 A1**
(43) Date of publication of application: **18.07.2007**
(21) Application number: 07000444.5
(22) Date of filing: 10.01.2007
(51) Int. Cl.: A61B 1/00, A61L 29/00

(54) **Endoscope**

(30) Priority: 13.01.2006 JP 2006006531
(71) Applicant: Fujinon Corporation, Saitama-shi, Saitama (JP)
(72) Inventor: Watanabe, Joji, Saitama-shi, Saitama (JP)
(74) Representative: Klunker . Schmitt-Nilson . Hirsch

(57) **Abstract**

An endoscope comprises: a rubber member having a slide area with another member constituting the endoscope, wherein polyalkylsiloxane is coated over the slide area of the rubber member.

## Description

### Background of the Invention

### 1. Filed of the Invention

The present invention relates to endoscopes, and more particularly to a medical endoscope having rubber members including, particularly, a plug, a valve's seal member and an insertion part's break-preventive member.

### 2. Description of the Related Art

The medical endoscope is an instrument including an operation part the operator is to grip and an insertion part provided continuous with the operation part so that, by inserting it at the insertion part into the patient's body, the body interior can be observed.

The endoscopic operation part is provided therein with a forceps-insert port. By inserting a treatment tool such as forceps through the forceps-insert port, the treatment tool can be drawn out of a forceps-exit port provided at the tip of the insertion part.

A mouthpiece is formed in the forceps-insert port so that a rubber-make plug can be attached at the tip of the mouthpiece. JP-A-2004-141303 discloses a plug structured with a plug body having an opening (treatment-tool insert port) and a cap for closing the opening. When inserting a treatment tool, the cap is removed from the plug body. When not inserting a treatment tool, it is fit to the plug body in order to prevent the reverse flow of humors, etc. The cap and the plug body are firmly tightened together not to be easily opened under the pressure of from the body interior. For this reason, there is a considerable difficulty in attaching and removing the cap to and from the plug body, thus resulting in a possibility that the cap is not perfectly closed.

In order to eliminate such a disadvantage, JP-A-11-253395 describes a plug that an ethylene-tetrafluoride resin is coated at an opening in the plug body so that the cap can be attached with smoothness. Meanwhile, JP-UM-A-62-155802 describes a plug manufactured by containing oil in rubber, thus improving lubricity.

However, the plug in JP-A-11-253395 is coated with an ethylene-tetrafluoride resin over the main body formed of an elastic material. Thus, there is involved a disadvantage that by repeating the engagement/disengagement of the cap, the coating is pealed off thus making it difficult to engage/disengage the cap. Furthermore, in the plug in JP-A-11-253395, the cap is made easier to attach by such coating. On the contrary, there is a disadvantage that the cap is to be easily removed from the plug body. Meanwhile, in the plug in JP-UM-A-62-155802, there is a problem that the oil ingredient is removed during cleaning resulting in lowered lubricity.

In this manner, the related-art plugs involve problems that either one of slidability or contact property can be satisfied and that proper slidability and contact property could not be kept over a long term. Such problems encounter in other endoscopic rubber members.

For example, the endoscopic operation part is provided with an air/water valve for supplying air/water to the tip of the insertion part and a vacuum valve for suction under pressure at the tip of the insertion part. Those valves use rubber-make seal members to seal the gap of between the piston and the cylinder. The seal members also require to maintain both the slidability and contact property well over a long term, similarly to the plug member. JP-A-9-122069 discloses a seal member in a form that is easy to deform but capable of preventing excessive deformation, as a seal member satisfying both slidability and contact property at the same time. However, in the seal member of JP-A-9-122069, there is a disadvantage of a difficulty in providing slidability and contact property in respective proper ranges because sealing is done by the elastic force thereof and that the elastic force decreases through use over a long term thus spoiling the contact property.

The endoscope includes a break-preventive member as another rubber member (see JP-UM-A-2-126601, for example). The break-preventive member is for preventing a non-rigid tube (e.g. flexible insertion part or universal cable) from bending with small radius-of-curvature by being sheathed over the non-rigid tube. The break-preventive member is necessarily placed in close contact with the outer peripheral surface of the non-rigid tube in order to ensure air-tightness. On the other hand, in order to protect the non-rigid tube, there is a need to allow the relevant member to slide over the non-rigid tube when the non-rigid tube is curved. For this reason, JP-UM-A-2-126601 discloses a break-preventive member processed for hydrophilic lubricity. However, in the break-preventive member of JP-UM-A-2-126601, the coating is pealed off upon elastic deformation of the break-preventive member, thus involving a problem that there is a difficulty in maintaining the slidability and contact property both well over a long term.

As described above, the endoscope uses a multiplicity of rubber members. Those rubber members require well slidability and contact property at the same time. However the related-art rubber members involve a problem that slidability and contact property are not maintained well over a long term.

### Summary of the Invention

The present invention has been made in view of such a circumstance, and it is an object thereof to provide an endoscope having a rubber member capable of maintaining both well slidability and contact property.

According to a first aspect of the invention, there is provided an endoscope comprising: a rubber member having a slide area with another member constituting the endoscope, wherein polyalkylsiloxane is coated over the slide area of the rubber member.

According to the first aspect of the invention, because the rubber member is coated at its sliding area with polyalkylsiloxane having a friction-reducing effect, friction resistance on the sliding area can be reduced while improving the slidability of the rubber member. Meanwhile, polyalkylsiloxane has a high contact property, thus having a property easy to deform but not readily stripped off when coated over an elastic member. Thus, according to the first aspect of the invention, the rubber member coated with polyalkylsiloxane is allowed to maintain contact property and slidability well over a long term.

According to a second aspect of the invention, there is provided the endoscope as set forth in the first aspect of the invention, further comprising an operation part including a treatment-tool inlet, wherein the rubber member is a plug to be fit to the treatment-tool inlet. The plug is a member required to maintain slidability and contact property over a long term. By coating polyalkylsiloxane over the plug, the plug maintains well contact property and slidability over a long term.

According to a third aspect of the invention, there is provided the endoscope as set forth in the second aspect of the invention, wherein the plug comprises: a plug body that is fit to the treatment-tool inlet and has an opening for receiving a treatment tool; a connection; and a cap member that is connected with the plug body through the connection and is fit to the plug body so as to close the opening, wherein at least one of the plug body and the cap member has the slide area coated with polyalkylsiloxane, the slide area being an area where sliding upon fitting of the cap member with the plug body.

According to the third aspect of the invention, because polyalkylsiloxane is coated over an area where sliding upon fitting of among the plug member and the cap member, the cap member can be easily fit to the plug member. Meanwhile, because rubber is left exposed in an area where sliding upon removal-without coating polyalkylsiloxane, friction resistance increases at between the plug body and the cap member, thus preventing the cap member from being removed from the plug body.

According to a fourth aspect of the invention, there is provided the endoscope as set forth in the first aspect of the invention, further comprising an operating part including a cylinder and a piston of a valve, wherein the rubber member is a seal member that seals a gap of between the cylinder and the piston.

According to the fourth aspect of the invention, because polyalkylsiloxane is coated over the seal member that seals the gap of between the cylinder and the piston, the piston and the cylinder can be kept in close contact (air-tight) while maintaining the slidability at between the piston and the cylinder. Incidentally, the endoscopic valves includes a feed-air/water valve to supply air/water to the tip of an insertion part and a vacuum valve to perform a vacuum operation at the tip of the insertion part.

According to a fifth aspect of the invention, there is provided the endoscope as set forth in the first aspect of the invention, further comprising a flexible tube, wherein the rubber member is a break-preventive member sheathed over the flexible tube and prevents the flexible tube from being broken.

According to the fifth aspect of the invention, because polyalkylsiloxane is coated over the break-preventive member, slidability can be improved at between the break-preventive member and the flexible tube while maintaining the break-preventive member in close contact with the flexible tube. Furthermore, such contact property and slidability can be kept over a long term. Incidentally, the flexible tubes the break-preventive member is to be sheathed over include a flexible insertion part to insert in the body interior of the patient and a flexible universal cable to be connected to the endoscopic operation part.

### Brief Description of the Drawings

Fig. 1 is a perspective view showing an endoscope to which the present invention is applied;
Fig. 2 is a perspective view showing an insertion part tip of the endoscope;
Fig. 3 is an assembly view of an operation part;
Fig. 4 is a sectional view of a forceps receiver;
Fig. 5 is a sectional view of a plug;
Fig. 6 is a sectional view of the plug;
Fig. 7 is a side view showing a feed-air/water button unit;
Fig. 8 is a fragmentary sectional view showing the feed-air/water button unit; and
Fig. 9 is a sectional view showing a break-preventive member.

### Detailed Description of the Invention

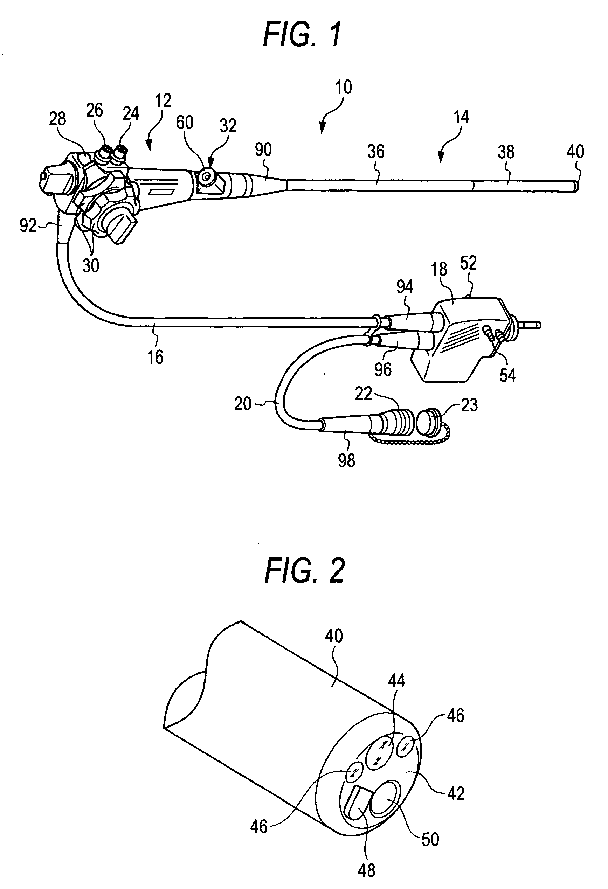

In accordance with the attached drawings, description will now be made on a preferred embodiment according to the present invention. Fig. 1 is a perspective view showing an endoscope to which the present invention is applied.

As shown in the figure, an endoscope 10 includes an operation part 12 and an insertion part 14 provided coupled to the operation part 12. The operation part 12 is connected with a universal cable 16 so that an LG connector 18 can be provided at the tip of the universal cable 16. The LG connector 18 is removably connected to a light source, not shown, to thereby convey illumination light to an illumination window (see Fig. 2), referred later. Meanwhile, the LG connector 18 is connected with an electric connector 22 through a cable 20 so that the electric connector 22 can be removably connected to a processor, not shown. Incidentally, reference numeral 23 shown in Fig.1 designates a cap for the electric connector 22, which is attached to the electric connector 22 when performing a cleaning.

In the operation part 12, a feed-air/water button 24, a vacuum button 26 and a shutter button 28 are arranged side by side, wherein a pair of angle knobs 30, 30 are provided. Meanwhile, a forceps receiver 32 is provided in the operation part 12 so that a plug 60, referred later, can be fit to the tip of the forceps receiver 32.

The insertion part 14 is made up with a non-rigid portion 36, a bendable portion 38 and a tip portion 40, in the closer order to the operation part 12. The bendable portion 38 can be operated remotely by rotating the angle knobs 30, 30 of the operation part 12. This allows the tip portion 40 to orient in a desired direction.

The tip portion 40 has its surface 42 where there are provided a viewing window (cover glass) 44, illumination windows 46, 46, a feed-air/water nozzle 48 and a forceps port 50.

In rear of the viewing window 44, a viewing optical system and a CCD (not shown) are arranged. The CCD is supported by a board connected with a signal cable (not shown). The signal cable is passed through the Fig. 1 insertion part 14, operation part 12, universal cable, etc. and extended to the electric connector 22 where connected to a processor, not shown. The observation image captured through the view window is focused on an image plane of the CCD and converted into an electric signal. The electric signal is outputted through the signal cable to the processor where it is converted into a video signal. This allows the monitor, (not shown) connected to the processor, to display an observation image.

In rear of the Fig. 2 illumination windows 46, 46, there is arranged a light-exit end of a light guide (not shown). The light guide passes through the Fig. 1 insertion part 14, operation part 12 and universal cable 16,-to have a light-incident end arranged in the LG connector 18. Accordingly, by connecting the LG connector 18 to a light source (not shown), the light illuminated from the light source is conveyed through the light guide to the illumination windows 46, 46 and radiated frontward through the illumination windows 46, 46.

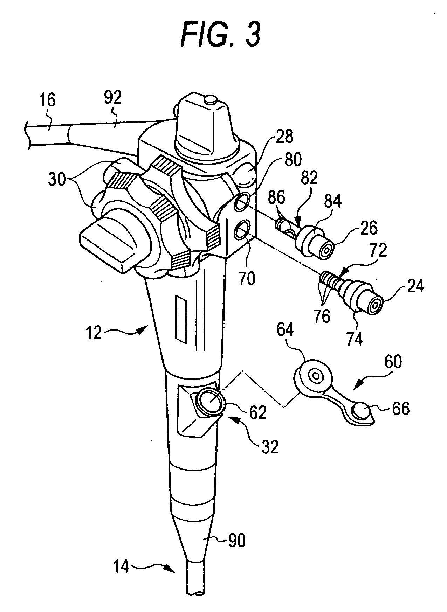

The air/water nozzle 48 in Fig. 2 communicates with a Fig. 3 air/water valve (cylinder 70 and piston 72) that is operable by means of a Fig. 3 feed-air/water button 2. The air/water valve communicates with an air/water connector 52 of the Fig. 1 LG connector 18. The air/water connector 52 is connected with a not-shown feed-air/water means so that air and water is to be supplied from the feed-air/water means. By operating the feed-air/water button 24, air or water can be ejected from the air/water nozzle 48 toward the viewing window 44.

The Fig. 2 forceps port 50 communicates with the Fig. 1 forceps receiver 32. By inserting a treatment tool such as forceps through the forceps receiver 32, the treatment tool can be drawn out of the forceps port 50. Meanwhile, the forceps port 50 communicates with a vacuum valve (cylinder 80 and piston 82) that is operable by means of the Fig. 3 vacuum button 26. The vacuum valve is further connected to a vacuum connector 54 of the Fig. 1 LG connector 18. By connecting a not-shown vacuum pump to the vacuum connector 54 and operating the vacuum valve by means of the vacuum button 26, a pathological tissue, etc. can be taken-under vacuum through the forceps port 50.

In the meanwhile, in the endoscope 10, a plurality of rubber members are used requiring well slidability and contact property at the same time. The rubber members are explained on an example with a plug 60.

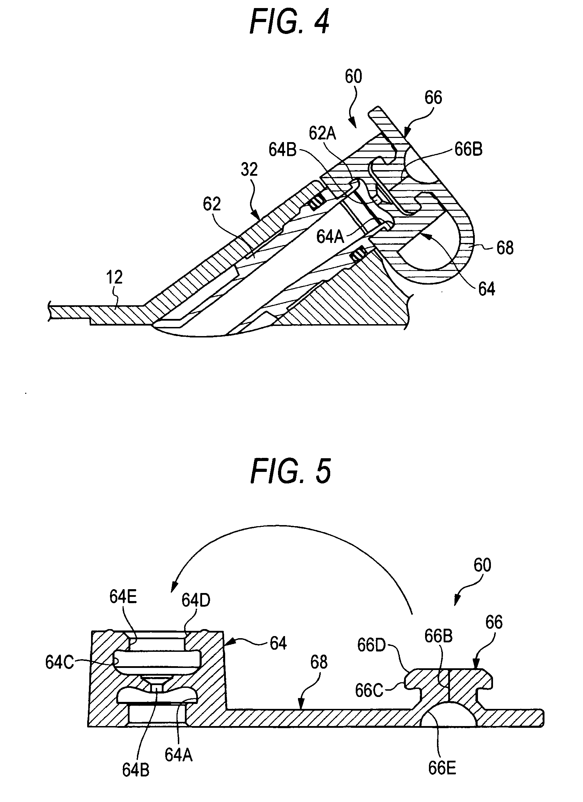

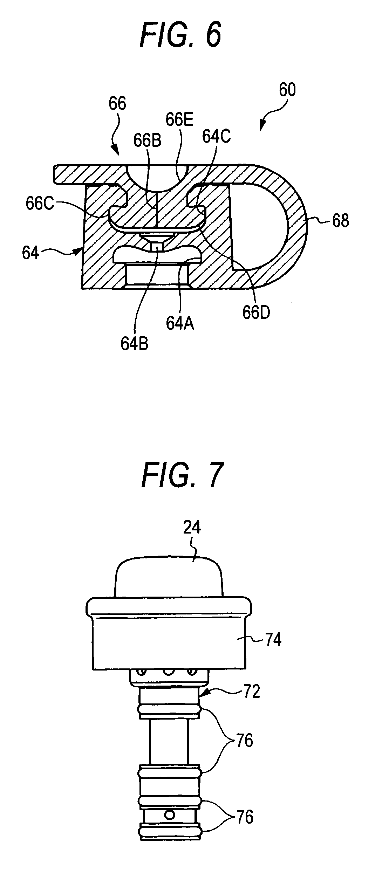

Fig. 3 shows an assembly view of the operation part 12. Fig. 4 is a sectional view showing a structure of the forceps receiver 32 while Figs. 5 and 6 are sectional views showing a plug 60. Fig. 5 shows a state that a cap 66 is removed from a plug body 64 while Fig. 6 shows a state that the cap 66 is attached to the plug body 64.

The forceps receiver 32 has a mouthpiece 62 fixed in the operation part 12, as shown in Fig. 4. The plug 60 is to be fit to the mouthpiece 62. The plug 60 is structured by connecting the plug body 64 and the cap 66 through a strip-formed connection 68, as shown in Fig. 5. Those of the plug body 64, the cap 66 and the connection 68 are integrally formed of a rubber material, such as silicone rubber. In this case, for any one member of the plug body 64 and the cap 66, the other corresponds to the other member. Incidentally, although the plug body 64, the cap 66 and the connection 68 need not be in one body, at least one of the plug body 64 and the cap 66 is formed of a rubber material over which rubber material is covered over with polyalkylsiloxane, referred later.

The plug body 64 is formed nearly cylindrical, thus forming a concave groove 64A over the entire circumference thereof. The groove 64A is formed such that the mouthpiece 62 at its flange 62A fits therewith. Accordingly, by fitting the flange 62A of the mouthpiece 62 in the groove 64A while elastically deforming the plug body 64, the plug body 64 is fixed on the mouthpiece 62. Incidentally, the fit force of between the plug body 64 and the mouthpiece 62 is provided greater than the fit force of between the plug body 64 and the cap 66.

The plug body 64 is formed with a restriction passage 64B in the inner peripheral surface thereof. The restriction passage 64B is provided for insertion of an endoscopic treatment tool, such as a forceps, and hence formed somewhat smaller in diameter than the endoscopic treatment tool. An annular concave 64C is formed entirely circumferentially on the side opposite to the groove 64A with respect to the restriction passage 64B. The annular concave 64C is formed to fit with the annular convex 66C of the cap 66.

The cap 66 is formed nearly in a circular cylindrical form, thus being formed entirely circumferentially with the annular convex 66C in the outer peripheral surface at one end thereof. As shown in Fig. 6, by bending the connection 68 and fitting the annular convex 66C of the cap 66 in the annular concave 64C of the plug body 64, the cap 66 is fit to the plug body 64.

Incidentally, in the cap 66, a taper 66D is formed in the outer periphery at a tip thereof (i.e. in the end on the side to be fit in the plug body 64). Meanwhile, in the plug body 64, a slant surface 64D is formed in the inner periphery at the tip thereof (i.e. in the end on the side the cap 66 is to fit), thus providing an inner diameter increasing as the tip is neared. This facilitates the fitting of between the cap 66 and the plug body 64.

A slit 66B is formed in the cap 66, at a central region thereof. Even in the state the cap 66 is fit on the plug body 64, a treatment tool having a small diameter, e.g. an injection needle, can be inserted through the slit 66B.

Incidentally, a semispherical cavity 66E is formed in the cap 66, which acts as a guide for inserting a small-diametered treatment tool into the slit 66B.

In the case of not using an endoscopic treatment tool such as a forceps for the plug 60 structured as above, the cap 66 is fit to the plug body 64. In such a case, the cap 66 is pushed in the plug body 64 while bending the connection 68 and elastically deforming the plug body 64, to thereby fit the annular convex 66C of the cap 66 in the annular concave 64C of the plug body 64. This places the cap 66 into fitting with the plug body 64, to close the restriction passage 64B by means of the cap 66. Thus, humors, etc. are prevented from flowing out-of the mouthpiece 62.

When the cap 66 is fit to the plug body 64, slide occurs partially between the cap 66 and the plug body 64. The cap 66 slides at its outer peripheral surface of the taper surface 66D and annular convex 66C while the plug body 64 slides at its slant surface 64D and at the inner peripheral surface 64E of between the slant surface 64D and the annular concave 64C. Those surfaces (66D, 66C, 64D, 64E) are collectively referred to as slide-upon-fitting surfaces.

The slide-upon-fitting surface is coated with polyalkylsiloxane. The means of coating is not limited but can use spraying, dipping, brushing, spin-coat or the like. After coating, baking is preferably done at 100 °C or higher (preferably at between °C 100 and 150°C). This improves the contact property of the polyalkylsiloxane to the slide-upon-fitting surface.

Polyalkylsiloxane has a friction-reducing effect. By coating polyalkylsiloxane over the slide-upon-fitting surface, it is possible to reduce the friction resistance caused by the sliding of between the cap 66 and the plug body 64. This can easily fit the cap 66 to the plug body 64. Meanwhile, because of a reduced frictional resistance caused by fitting the cap 66 to the plug body 64, contact property can be improved by reducing the tolerance of between the cap 66 and the plug body 64. This can improve the air-tightness in fitting the cap 66 on the plug body 64.

Meanwhile, because the coating of polyalkylsiloxane has a polymeric structure having a good contact property to its base material, it provides a well contact property to the cap 66 and to the plug body 64 that are elastic members. When the cap 66 or the plug body 64 is elastically deformed, the coating of polyalkylsiloxane thereon is to deform together therewith. Accordingly, even where the cap 66 is repeatedly attached to and removed from the plug body 64, the coating of polyalkylsiloxane is not easily pealed off, thus maintaining slidability and contact property over a long term.

Meanwhile, where polyalkylsiloxane is coated, there is no need of pre-treatment thus realizing a coating at low cost. Accordingly, the coating of polyalkylsiloxane, if applied, can provide at low coat a lubricity well in durability without spoiling the flexibility of rubber, as compared to the case of coating with another coat material.

Furthermore, because the coating of polyalkylsiloxane can be maintained over a long term, it is possible to prevent the occurrence of blooming that is ready to occur upon repeatedly attaching and removing the cap 66 to and from the plug body 64.

As for another rubber member, explanation is now made on an example of a seal member of an air/water valve provided in the operation part 12.

The air/water valve is structured with a cylinder 70 and a piston 72 that are shown in Fig. 3. The cylinder 70 is fixed in the operation part 70 while the piston 72 is removably provided in the cylinder 70. The piston 72 is attached with a feed-air/water button 24 at its upper end. Meanwhile, the piston 72 is provided thereon with a knob member 74 so that the piston 72 can be held for axial sliding relative to the knob member 74. The knob member 74 is structured to be fit to the operation part 12. The piston 72, the feed-air/water button 24 and the knob member 74 are unitized as a feed-air/water unit that can be removably attached to the cylinder 70.

Fig. 7 is a side view showing a feed-air/water button unit. As shown in the figure, the piston 72 is fit thereon with a plurality of O-rings (corresponding to seal members) 76, 76 ... formed of a rubber member, such as of silicone rubber, fluorinated rubber or EPDM. When the feed-air/water button unit is attached to the cylinder 70, the O-rings 76 are arranged between the outer peripheral surface of the piston 72 and the inner peripheral surface of the cylinder 70, thereby ensuring the air-tightness at between the piston 72 and the cylinder 70. Accordingly, close contact property is required for both the pistons 72 and the cylinder 70. Meanwhile, when the feed-air/water button 24 is operated, the O-rings 76 move together with the piston 72 and slide over the cylinder 70. Accordingly, slidability is also required for those.

For this reason, polyalkylsiloxane is coated over the surface of the O-rings 76. Polyalkylsiloxane has a property not easy to be stripped off because having a friction-reducing effect and well contact property as noted before. Accordingly, slidability can be enhanced at between the O-rings 76 and the cylinder 70 while ensuring air-tightness (contact property) due to the O-rings 76. Furthermore, such air-tightness and slidability can be maintained well over a long term.

Meanwhile, by enhancing the slidability between the O-rings 76 and the cylinder 70 through use of polyalkylsiloxane, the force is reduced that is required in pressing the feed-air/water button 24, thus improving the operationality of the valve. Furthermore, the coating of polyalkylsiloxane ensures the positive slide between the O-rings 76 and the cylinder 70. This can reduce the compression force of a spring used to return the piston 72 to the former position.

As for another rubber member, explanation is now made on an example of a seal member of a vacuum valve provided in the operation part 12.

The vacuum valve is structured with a cylinder 80 and a piston 82 that are shown in Fig. 3. The cylinder 80 is fixed in the operation part 12 so that the piston 82 can be removably provided in the cylinder 80. The piston 80 is provided with a vacuum button 26 at the upper end thereof, and with a knob member 84. The piston 82 is held axially slidably in the knob member 84. The knob member 84 is formed to be fit to the operation part 12. The piston 82, the vacuum button 26 and the knob member 84 are unitized as a vacuum button unit that is removably attached in the cylinder 80.

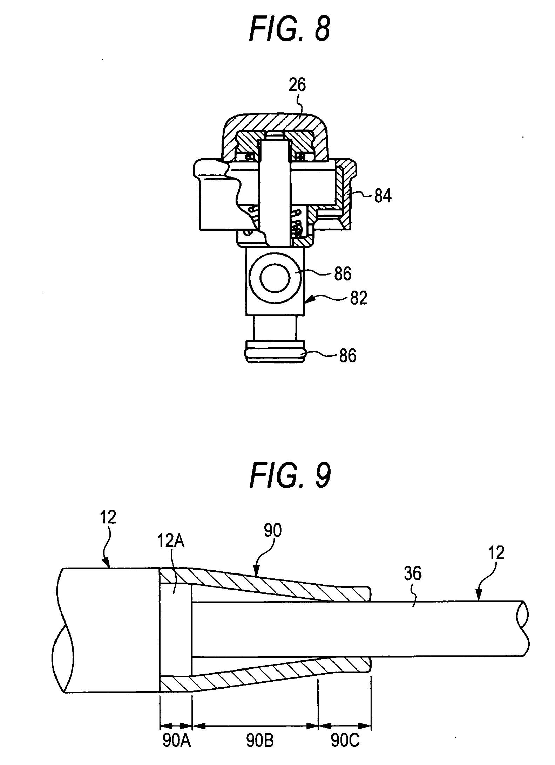

Fig. 8 is a side view showing the vacuum button unit. As shown in the figure, the piston 82 is fit thereon with a plurality of O-rings (corresponding to seal members) 86, 86 ... formed of a rubber member, such as of silicone rubber, fluorinated rubber or EPDM. When the feed-air/water button unit is attached to the cylinder 70, the O-rings 86 are arranged between the outer peripheral surface of the piston 82 and the inner peripheral surface of the cylinder 8, thereby keeping the air-tightness at between the piston 82 and the cylinder 80. Accordingly, close contact property is required for both the pistons 82 and the cylinder 80. Meanwhile, when the feed-air/water button 24 is operated, the O-rings 86 move together with the piston 82 and slide over the cylinder 80. Accordingly, slidability also is also required for those.

For this reason, polyalkylsiloxane is coated over the surface of the O-rings 86. Polyalkylsiloxane has a property not easy to be stripped off because having a friction-reducing effect and well contact property as noted before. Accordingly, slidability can be enhanced at between the O-rings 86 and the cylinder 80 while ensuring air-tightness (contact property) due to the O-rings 86. Furthermore, such air-tightness and slidability can be maintained well over a long term.

Meanwhile, by enhancing the slidability between the O-rings 86 and the cylinder 80 through use of polyalkylsiloxane, the force is reduced that is required in pressing the feed-air/water button, thus improving the operationality of the valve. Furthermore, the coating of polyalkylsiloxane ensures the positive slide at between the O-rings 86 and the cylinder 80. This can reduce the compression force of a spring used to return the piston 82 to the former position.

Although the embodiment explained on the example with the feed-air/water valve and the vacuum valve, the invention is applicable also for the seal members of other valves used in the endoscope. For example, LG connector 18 shown in the Fig. 1 is provided with a valve communicating with the vacuum connector 54, the valve seal member may be coated with polyalkylsiloxane.

Now, a break-preventive member is explained as an example of another rubber member. The endoscope 10 uses a plurality of flexible tubes (e.g. the insertion part 14, the universal cable 16, the cable 20, etc.). In case such a flexible tube is bent at a small radius-of-curvature, there is a possibility to damage the interior content thereof (e.g. the light guide, the signal cable, the feed-air/water tube or the vacuum tube). For this reason, a rubber-make break-preventive member is sheathed for the purpose of protection.

For example, the non-rigid portion 36 of the insertion part 14 is sheathed with a break-preventive member 90 formed of rubber, such as of silicone rubber, fluorinated rubber or EPDM, at the end thereof closer to the operation part 12. The break-preventive member 90 has a connecting portion 90A formed cylindrical so that the connecting portion 90A can be fit and fixed over a connecting portion 12A of the operation part 12. The connecting portion 90A has, at its tip, a taper portion 90B continuously provided nearly in a headless conical form having a diameter decreasing as the tip is neared. The taper portion 90B has, at its tip, a straight portion 90C continuously provided in a cylindrical form. The straight portion 90C is formed with an inner diameter somewhat smaller than the outer diameter of the non-rigid portion 36 of the insertion part 14. When the non-rigid portion 36 is inserted through the straight portion 90C, the straight portion 90C is placed, at its inner peripheral surface, in close contact with the outer peripheral surface of the non-rigid portion 36. This can keep air-tight the interior of the operation part, thus preventing humors or foreign matters from intruding into the operation part 12.

The break-preventive member 90 is integrally formed of a rubber material such as a silicone rubber, at its connecting portion 90A, taper portion 90B and straight portion 90C. Polyalkylsiloxane is coated over the inner peripheral surface of the straight portion 90C. Accordingly, the straight portion 90C of the break-preventive member 90 has a reduced friction resistance with the non-rigid portion 36, thus providing a superior slidability with the non-rigid portion 36.

With the break-preventive member 90 thus structured, when the non-rigid portion 36 is bent greatly, the straight portion 90C elastically deforms while being closely contacted with the non-rigid portion 36. The stress applied to the non-rigid portion 36 is dispersed thus preventing the non-rigid portion 36 from breaking. On this occasion, in the absence of a coating of polyalkylsiloxane, the straight portion 90C will not slide over the non-rigid portion 36, thus resulting in a possibility to cause a crack in the non-rigid portion 36 by a great load acting upon the break-preventive member 90 or the non-rigid portion is damaged by a great stress applied to the non-rigid portion 36. In this embodiment, because polyalkylsiloxane is coated over the straight portion 90C of the break-preventive member 90, the straight portion 90C is well slidable over the non-rigid portion 36 while keeping air-tightness thus preventing the break-preventive member 90 and the non-rigid portion 36 from being damaged.

Meanwhile, the coating of polyalkylsiloxane has a well contact property to but not readily pealed off the break-preventive-member 90 made by a rubber member. Thus, the break-preventive member 90 can maintain well the slidability over and contact property to the non-rigid portion 36 over a long term.

Incidentally, although the foregoing embodiments explained on the example the invention is applied to the break-preventive member 90 for the non-rigid portion 36 of the insertion part 14, the invention may be applied to another break-preventive member. Namely, although the endoscope 10 shown in Fig. 1 is provided with break-preventive members 92, 94 for the universal cable 16 and break-preventive members 96, 98 for the cable 20, the invention may be applied to those break-preventive members 92, 94, 96, 98. For those break-preventive members 92, 94, 96, 98, by coating polyalkylsiloxane over the slide surfaces with the universal cable 16 and cable 20, well slidability can be obtained while keeping a well contact property to the universal cable 16 and cable 20. Such contact property and slidability can be maintained well over a long term.

Meanwhile, the rubber member the invention is applied is not limited to those of the foregoing embodiments but the invention can be applied provided that the relevant rubber member has a slide portion in the endoscope 10. For example, the cap 23 of the Fig. 1 electrical connector 22 may be formed of a rubber material and polyalkylsiloxane be coated over the slide portion thereof with the electric connector 22.

Meanwhile, polyalkylsiloxane may be coated over the interior--content of the insertion part 14 or universal cable 16 (a light guide, a signal cable, freed-air/water tube, a vacuum tube, etc). Namely, where the light guide or the signal cable is coated with a rubber material such as a silicone rubber, polyalkylsiloxane is desirably coated over the coating. Where the feed-air/water tube or the vacuum tube is coated with a rubber material such as a silicone rubber, polyalkylsiloxane is desirably coated over the outer surface thereof. This can reduce the frictions of between the interior contents, thus preventing the damage to the interior contents as caused upon curving the insertion part 14 or the universal cable 16.

According to the present invention, polyalkylsiloxane is coated over a slide area of a rubber member for the endoscope. Accordingly, slidability and contact property can be both obtained well, wherein such slidability and contact property can be maintained over a long term.

The entire disclosure of each and every foreign patent application from which the benefit of foreign priority has been claimed in the present application is incorporated herein by reference, as if fully set forth.

## Claims

1. An endoscope comprising:
a rubber member having a slide area with another member constituting the endoscope,
wherein polyalkylsiloxane is coated over the slide area of the rubber member.

2. An endoscope according to claim 1, further comprising an operation part including a treatment-tool inlet,
wherein the rubber member is a plug to be fit to the treatment-tool inlet.

3. An endoscope according to claim 2,
wherein the plug comprises:
a plug body that is fit to the treatment-tool inlet and has an opening for receiving a treatment tool;
a connection; and
a cap member that is connected with the plug body through the connection and is fit to the plug body so as to close the opening,
wherein at least one of the plug body and the cap member has the slide area coated with polyalkylsiloxane, the slide area being an area where sliding upon fitting of the cap member with the plug body.

4. An endoscope according to claim 1, further comprising an operating part including a cylinder and a piston of a valve,
wherein the rubber member is a seal member that seals a gap of between the cylinder and the piston.

5. An endoscope according to claim 1, further comprising a flexible tube,
wherein the rubber member is a break-preventive member sheathed over the flexible tube and prevents the flexible tube from being broken.
